Europäisches Patentamt

European Patent Office

Office européen des brevets

(11) Publication number: **0 117 883**
**B1**

(12) **EUROPEAN PATENT SPECIFICATION**

(45) Date of publication of patent specification: **28.01.87**

(51) Int. Cl.⁴: $C\ 07\ D\ 239/36$

(21) Application number: **83102157.1**

(22) Date of filing: **04.03.83**

(54) Preparation of 2-t-butyl-5-hydroxypyrimidine by hydrolysis of 2-t-butyl-5--bromo/chloropyrimidine.

(43) Date of publication of application:
**12.09.84 Bulletin 84/37**

(45) Publication of the grant of the patent:
**28.01.87 Bulletin 87/05**

(84) Designated Contracting States:
**BE CH DE FR GB IT LI NL**

(56) References cited:

D.J. BROWN: "The pyrimidines", supplement I,
Interscience chapter VI, section 5, paragraph E,
1970, page 148, Wiley-Interscience, New York,
US

The file contains technical information
submitted after the application was filed and
not included in this specification

(73) Proprietor: **THE DOW CHEMICAL COMPANY**
**2030 Dow Center Abbott Road P.O. Box 1967**
**Midland, MI 48640 (US)**

(72) Inventor: **Pews, Richard Garth**
**4403 Andre**
**Midland Michigan 48640 (US)**

(74) Representative: **Weickmann, Heinrich, Dipl.-Ing.**
**et al**
**Patentanwälte Dipl.-Ing. H.Weickmann Dipl.-**
**Phys.Dr. K.Fincke Dipl.-Ing. F.A.Weickmann**
**Dipl.-Chem. B. Huber Dr.-Ing. H. Liska Dipl.-**
**Phys.Dr. J. Prechtel Postfach 860820**
**D-8000 München 86 (DE)**

Courier Press, Leamington Spa, England.

# 0 117 883

**Description**

In "The Pyrimidines", Interscience (1962), pages 203—204, it is stated that, "The conversion of an active halogeno into an hydroxy group is rather uncommon, and not always easy". In Supplement I of "The Pyrimidines", Interscience (1970), page 148, there is the statement, "The direct hydrolysis of a chloro- to an hydroxy-pyrimidine was avoided for many years, but no such inhibition seems to be operating now. Acid or alkali may be used, although the latter is more usual, . . . ." However, attempts to hydrolyze 2-t-butyl-5-bromopyrimidine with sodium methoxide gave considerable quantities of byproduct 2-t-butyl-pyrimidine.

It has now been found that the hydrolysis of 2-t-butyl-5-halopyrimidines to prepare 2-t-butyl-5-hydroxypyrimidine in good yields and purity may be carried out in the presence of an alkali metal methoxide and a catalytic amount of an N-oxide, a disulfide or elemental sulfur.

The reaction is advantageously carried out at temperatures of from 100° to 300°C, preferably 150° to 220°C and most advantageously at 160° to 180°C at ambient pressure in a Parr bomb, in the presence of from 0.5 to 20 mole percent of catalyst. The preferred catalyst is elemental sulfur and it is preferably employed in an amount of from 2.5 to 7.5 mole percent. Sodium methoxide is preferably employed, although other alkali metal methoxides such as potassium methoxide may be employed, if desired.

The hydrolysis is advantageously and preferably carried out in a methyl alcohol solvent.

The invention is further illustrated by the following Examples, wherein 2-t-butyl-5-bromo(or chloro*)-pyrimidine was hydrolyzed in the presence of sodium methoxide in excess methanol in a Parr bomb. The following products were obtained in the amounts indicated in Table I (parts by weight):

A. 2-t-butylpyrimidine
B. 2-t-butyl-5-methoxypyrimidine
C. 2-t-butyl-5-hydroxypyrimidine

TABLE I

| Run | Catalyst | Mole % | Temp. °C | Products A | B | C |
|-----|----------|--------|----------|---|---|---|
| 1 | None | 0 | 150 | 23 | 11 | 77 |
| 2 | None | 0 | 150 | 37 | 11 | 58 |
| 3 | 2-Picoline-N-oxide | 10 | 152 | 12 | 12 | 69 |
| 4 | 2-Picoline-N-oxide | 20 | 152 | 12 | 1 | 76 |
| 5 | Di-n-butyl-disulfide | 5 | 160 | 12 | 1 | 91 |
| 6 | D-n-butyl-disulfide | 10 | 160 | 7 | 1 | 105 |
| 7 | S° | 5 | 163 | 4 | 12 | 88 |
| 8 | S° | 5 | 165 | 5 | 1 | 94 |
| 9 | S° | 2.5 | 153 | 7 | 15 | 80 |
| 10 | S° | 7.5 | 156 | 4 | — | 100 |
| 11 | S° | 5 | 155 | 8 | 4 | 93 |
| 12 | S° | 2.5 | 155 | 7 | 2 | 93 |
| 13 | S° | 1.5 | 160 | 10.14 | — | 101.41 |
| 14 | S° | 0.5 | 160 | 12.06 | — | 97.55 |
| 15* | S° | 2.5 | 170 | 4 | — | 100 |

*Hydrolysis of 2-t-butyl-5-chloropyrimidine.

It has further been found that 2-t-butyl-5-chloropyrimidine may be made in good yields and purity by the direct chlorination of 2-t-butylpyrimidine with elemental chlorine in an acetic or propionic acid solution at temperatures of 40° to 90°C, and preferably in the presence of a buffer.

2

The chlorination of 2-t-butylpyrimidine is preferably carried out at temperatures of 55° to 65°C and in the absence of a catalyst. The solution of 2-t-butylpyrimidine preferably contains from 1.5 to 3.0 moles pyrimidine per liter of solvent, although higher or lower amounts may be employed if desired. In more dilute solutions, greater energy requirements must be met in order to separate the desired product and chlorination of the solvent itself is more apt to take place. In more concentrated solutions, it is more difficult to maintain a uniform mixture or solution because of increased viscosity.

Suitable buffers include, for example, sodium acetate, potassium acetate, sodium propionate and potassium propionate. It is desirable that the buffer be soluble in the solvent. Sodium acetate is preferred.

The preferred solvent is acetic acid.

The chlorination reaction is further illustrated by the examples according to Table II.

TABLE II

| Run | t-Butyl-pyrimidine (Moles) | Acetic Acid (ml) | Sodium Acetate (Moles) | Temperature (°C) | Time (hrs) | Yield (%) |
|---|---|---|---|---|---|---|
| 1 | 0.022 | 25 | 0.026 | 60 | 4.5 | 97 |
| 2 | 0.367 | 250 | 0.44 | 60 | 6.0 | 75 |
| 3 | 0.734 | 500 | 0.88 | 60 | 6.0 | 86 |
| 4 | 0.734 | 500 | 0.88 | 60 | 6.0 | 97 |
| 5 | 0.734 | 500 | 0.88 | 60 | 6.0 | 73 |
| 6 | 0.1 | 68 | 0.12 | 60 | 3.5 | 83 |
| 7 | 1.47 | 500 | 1.76 | 60 | 13.0 | 79 |
| 8 | 1.47 | 500 | 1.76 | 60 | 10.5 | 82 |

Similar results may be obtained when employing propionic acid as the solvent and/or utilizing other buffers such as, for example, potassium acetate, sodium propionate and potassium propionate.

**Claims**

1. A method of making 2-t-butyl-5-hydroxypyrimidine which comprises hydrolyzing a 2-t-butyl-5-halopyrimidine in the presence of an alkali metal methoxide and a catalyst comprising an N-oxide, a disulfide or elemental sulfur.

2. Method of Claim 1 wherein the halopyrimidine is 2-t-butyl-5-bromopyrimidine.

3. Method of Claim 2 wherein the reaction is carried out at 150° to 220°C.

4. Method of Claim 2 wherein the catalyst is 2-picoline-N-oxide, di-n-butyldisulfide or elemental sulfur.

5. Method of Claim 1 wherein the halopyrimidine is 2-t-butyl-5-chloropyrimidine.

6. Method of Claim 5 wherein the 2-t-butyl-5-chloropyrimidine is prepared by chlorinating 2-t-butylpyrimidine in acetic or propionic acid solution with elemental chlorine in the absence of a catalyst at temperatures of from 40° to 90°C.

7. Method of Claim 6 wherein in the chlorination step the reaction temperature is from 55° to 65°C.

8. Method of Claim 7 wherein in the chlorination step the reaction is carried out for from 3 to 15 hours.

9. Method of Claim 6 wherein in the chlorination step the solvent is buffered with sodium acetate.

10. Method of Claim 9 wherein the solvent is acetic acid.

**Patentansprüche**

1. Verfahren zur Herstellung von 2-t-Butyl-5-hydroxypyrimidin, dadurch gekennzeichnet, daß man 2-t-Butyl-5-halogen-pyrimidin in Gegenwart eines Alkalimetallmethoxids und eines Katalysators, der ein N-Oxid, ein Disulfid oder elementaren Schwefel umfaßt, hydrolysiert.

2. Verfahren nach Anspruch 1, dadurch gekennzeichnet, daß das Halogenpyrimidin 2-t-Butyl-5-brompyrimidin ist.

3. Verfahren nach Anspruch 2, dadurch gekennzeichnet, daß die Reaktion bei 150° bis 220°C ausgeführt wird.

4. Verfahren nach Anspruch 2, dadurch gekennzeichnet, daß der Katalysator 2-Picolin-N-oxid, Di-n-butyldisulfid oder elementarer Schwefel ist.

3

**0 117 883**

5. Verfahren nach Anspruch 1, dadurch gekennzeichnet, daß das Halogenpyrimidin 2-t-Butyl-5-chloro-pyrimidin ist.

6. Verfahren nach Anspruch 5, dadurch gekennzeichnet, daß das 2-t-Butyl-5-chloro-pyrimidin hergestellt wird durch Chlorierung von 2-t-Butyl-pyrimidin in Essig- oder Propionsäure-Lösung mit elementarem Chlor in Abwesenheit eines Katalysators bei Temperaturen von 40° bis 90°C.

7. Verfahren nach Anspruch 6, dadurch gekennzeichnet, daß in der Chlorierungsstufe die Reaktionstemperatur 55° bis 65°C beträgt.

8. Verfahren nach Anspruch 7, dadurch gekennzeichnet, daß in der Chlorierungsstufe die Reaktion 3 bis 15 Stunden durchgeführt wird.

9. Verfahren nach Anspruch 6, dadurch gekennzeichnet, daß in der Chlorierungsstufe das Lösungsmittel mit Natriumacetat gepuffert ist.

10. Verfahren nach Anspruch 9, dadurch gekennzeichnet, daß das Lösungsmittel Essigsäure ist.

## Revendications

1. Procédé de fabrication de la 2-t-butyl-5-hydroxy-pyrimidine, qui comprend l'hydrolyse d'une 2-t-butyl-5-halopyrimidine, en présence d'un méthoxyde de métal alcalin et d'un catalyseur comprenant un N-oxyde, un disulfure ou du soufre élémentaire.

2. Procédé conforme à la revendication 1, dans lequel l'halopyrimidine est la 2-t-butyl-5-bromo-pyrimidine.

3. Procédé conforme à la revendication 2, dans lequel la réaction est effectuée à 150—220°C.

4. Procédé conforme à la revendication 2, dans lequel le catalyseur est le 2-picoline-N-oxyde, le di-n-butyl-disulfure, ou le soufre élémentaire.

5. Procédé conforme à la revendication 1, dans lequel l'halopyrimidine est la 2-t-butyl-5-chloro-pyrimidine.

6. Procédé conforme à la revendication 5, dans lequel on prépare la 2-t-butyl-5-chloropyrimidine en chlorant à l'aide de chlore élémentaire la 2-t-butylpyrimidine en solution dans de l'acide acétique ou de l'acide propionique, en l'absence de catalyseur, à des températures de 40—90°C.

7. Procédé conforme à la revendication 6, dans lequel la température de réaction est de 55—65°C au cours de l'étape de chloration.

8. Procédé conforme à la revendication 7, dans lequel on effectue la réaction de l'étape de chloration pendant 3 à 15 heures.

9. Procédé conforme à la revendication 6, dans lequel au cours de l'étape de chloration, le solvant est tamponné avec de l'acétate de sodium.

10. Procédé conforme à la revendication 9, dans lequel le solvant est l'acide acétique.

4